# EUROPÄISCHE PATENTSCHRIFT

(11) **EP 3 436 029 B1**
(45) Veröffentlichungstag und Bekanntmachung des Hinweises auf die Patenterteilung: **26.02.2020**
(21) Anmeldenummer: 17717093.3
(22) Anmeldetag: 24.03.2017
(51) Int. Cl.: A61K 31/7032, A61P 17/00, C07H 15/04, A61K 8/60, A61Q 19/00

(54) **DEXPANTHENOLGLYKOSID-ENTHALTENDE VERBINDUNG**
COMPOUND COMPRISING DEXPANTHENOLGLYCOSIDE
COMPOSÈ COMPRENANT UN GLYCOSIDE DE DEXPANTHENOL

(30) Priorität: 01.04.2016 DE 102016205474
(43) Veröffentlichungstag der Anmeldung: 06.02.2019
(73) Patentinhaber: Julius-Maximilians-Universität Würzburg, 97070 Würzburg (DE)
(72) Erfinder: SEIBEL, Jürgen, 97070 Würzburg (DE); TIMM, Malte, 97218 Gerbrunn (DE)
(74) Vertreter: FDST Patentanwälte
(86) Internationale Anmeldenummer: PCT/EP2017/057038
(87) Internationale Veröffentlichungsnummer: WO 2017/167652

(56) Entgegenhaltungen:
- EBNER FRITZ ET AL: "Topical use of dexpanthenol in skin disorders", AMERICAN JOURNAL OF CLINICAL DERMATO, ADIS, US, Bd. 3, Nr. 6, 1. Januar 2002 (2002-01-01), Seiten 427-433, XP009181863, ISSN: 1175-0561, DOI: 10.2165/00128071-200203060-00005

## Beschreibung

Die Erfindung betrifft eine Dexpanthenol enthaltende Verbindung, die insbesondere geeignet ist zur Verwendung in therapeutischen und/oder kosmetischen Verfahren.

Die menschliche Haut stellt das oberflächengrößte Organ des Menschen dar und dient als sogenanntes Hüllorgan des menschlichen Körpers. Die Haut übernimmt wichtige Funktionen im Bereich des Stoffwechsels und der Immunologie und verfügt über vielfältige Anpassungsmechanismen.

Die wichtigste Funktion der Haut ist ihre Barrierefunktion, also der Schutz des Organismus vor dem Austrocknen. Gesunde Haut benötigt normalerweise keinerlei Hilfsmittel zur Erhaltung der natürlichen Feuchtigkeit, da die Hautfeuchtigkeit durch natürliche Feuchthaltefaktoren, sogenannte "Natural Moisturizing Factors" (NMF), in der Epidermis, der obersten Hautschicht, reguliert wird, die bei der Neubildung der Haut stetig nachgebildet werden. Jedoch tragen Umweltbelastungen, ebenso wie trockene Luft, oder die Exposition von UV-Strahlung, dazu bei, dass der Haut Feuchtigkeit entzogen wird und/oder die natürlichen Feuchthaltefaktoren zerstört werden.

Eine mangelnde Versorgung der Haut mit Feuchtigkeit kann sich insbesondere mit fortschreitendem Alter beispielsweise durch Pigmentierung, Faltenbildung, Erschlaffung und Entzündungen der Haut zeigen. Bei trockener Haut können sich beispielsweise Falten und Mikrorisse bilden, in die Keime eindringen, welche dann in der Epidermis zu Hautinfektionen führen können.

Um dies zu verhindern bzw. um die negativen Auswirkungen zu minimieren, werden häufig Cremes und Lotionen mit Dexpanthenol als Wirkstoff eingesetzt. Dexpanthenol wird zur Hautpflege, ebenso wie zur topischen Behandlung von Hauterkrankungen verwendet. Aufgrund seiner hygroskopischen Eigenschaften verbessert Dexpanthenol die Feuchtigkeitsversorgung der Haut, verringert deren Rauheit und dient der Bekämpfung von Hautirritationen und somit der indirekten Linderung von Entzündungen. Als Bestandteil von Salben hat sich Dexpanthenol beispielsweise bei oberflächlichen Haut- und Schleimhautschäden bewährt, ebenso wie in Schaumsprays bei der Behandlung erstgradiger Verbrennungen. Weiter regt Dexpanthenol das Zellwachstum an und verringert so die Vernarbungszeit von Wunden.

Allerdings wird Dexpanthenol schnell von der Haut resorbiert und über den Blutkreislauf zu Pantothensäure, auch als Provitamin B5 bekannt, umgewandelt. Der Wirkzeitraum von Dexpanthenol nach Auftrag auf die Haut ist somit zeitlich begrenzt.

Aus F. Ebner, et al., "Topical Use of Dexpanthenol in Skin Disorders", Am J Clin Dermatol 2002, 3(6), S.427-433 ist bekannt, Dexpanthenol als Dermatikum einzusetzen. Hierin ist auch die schnelle Hautabsorption von Dexpanthenol und dessen Umwandlung in Pantothensäure beschrieben.

Der Erfindung liegt als eine Aufgabe zugrunde, den Wirkzeitraum von im Rahmen therapeutischer und/oder kosmetischer Verfahren auf die Haut aufgetragenem Dexpanthenol zu verlängern.

Diese Aufgabe wird erfindungsgemäß gelöst durch eine Verbindung, gekennzeichnet durch ein chemisch an ein Saccharid gebundenes Dexpanthenol.

In einem ersten Schritt berücksichtigt die Erfindung die Tatsache, dass Kosmetika, Cremes und auch Arzneimittel auf dem Markt erhältlich sind, bei denen der Wirkzeitraum durch eine zeitverzögerte Freisetzung der enthaltenen Wirkstoffe gesteuert wird. Dies wird zum Beispiel durch Umhüllungen, Einbettungen oder Mikroverkapselung der enthaltenen Wirkstoffe erreicht, da hierdurch die Wirkstofffreisetzung aus dem Hautpflegeprodukt oder dem Arzneimittel verlangsamt wird. Bei Hautpflegeprodukten kommen weiter Mehrfachemulsionen zum Einsatz, in welchen nebeneinander fettlösliche und wasserlösliche Wirkstoffe in die Haut geschleust und nacheinander freigesetzt werden. Die Wirkung kann durch die verzögerte Freigabe der Wirkstoffe entsprechend verlängert werden. Bei Arzneimitteln gibt es sogenannte retardierende Arzneiformen, mittels derer sich definierte zeitliche Verläufe der Arzneistofffreisetzung erzielen lassen.

In einem zweiten Schritt erkennt die Erfindung, dass sich eine verzögerte Freisetzung eines Wirkstoffs insbesondere dann erreichen lässt, wenn der Wirkstoff als ein Glycosid eingesetzt wird. Ein solches Glycosid wird nämlich, wie im Folgenden noch näher beschrieben werden wird, im oder am Körper erst allmählich unter Freisetzung des Wirkstoffs durch natürlicherweise vorhandene Enzyme gespalten. In einem dritten Schritt überträgt die Erfindung diese Erkenntnis auf den Wirkstoff Dexpanthenol, indem dieses nicht als isoliertes Molekül, sondern in Form eines Glycosids eingesetzt wird. Die erfindungsgemäß angegebene Verbindung ist durch ein chemisch an ein Saccharid gebundenes Dexpanthenol gekennzeichnet. Durch eine solche Modifikation von Dexpanthenol wird die gewünschte verzögerte Wirkstofffreisetzung von Dexpanthenol ermöglicht.

Wird die Verbindung, alleine oder als Bestandteil einer Zusammensetzung, auf die Haut aufgetragen, so wird das Dexpanthenol erst bei einer Spaltung der Verbindung, also zeitverzögert, freigesetzt. Somit wird auch Dexpanthenol als Wirkstoff erst nach erfolgter Bindungsspaltung wirksam. Mit anderen Worten wird der Wirkzeitraum des Dexpanthenols in Abhängigkeit der Abspaltung des chemisch gebundenen Saccharids verlängert.

Die Bindungsspaltung erfolgt vorzugsweise durch eine Reaktion der Verbindung mit einem oder mehreren Enzymen. Vorteilhafterweise wird die Verbindung in Anwesenheit von Glucosidasen gespalten. Glucosidasen sind Enzyme, die unter anderem von natürlicherweise auf der Haut vorkommenden Bakterien und Pilzen produziert werden. Nach Auftrag der Verbindung auf die Haut wird die Verbindung durch die von den Bakterien und/oder den Pilzen als Enzyme produzierten Glucosidasen unter Freisetzung von Dexpanthenol gespalten, mit anderen Worten also die Bindung zwischen dem Dexpanthenol und dem Saccharid aufgetrennt.

Zusätzlich weist die Verbindung des chemisch an ein Saccharid gebundenen Dexpanthenols nach eigenen Untersuchungen eine gegenüber reinem Dexpanthenol erhöhte Polarität auf. Hiermit einher geht eine verstärkte Hydratation, also eine Anlagerung von Wassermolekülen unter Ausbildung einer Hydrathülle. Als Folge ist eine gegenüber reinem Dexpanthenol verlangsamte Penetration in die Haut beobachtbar, was ein weiterer Vorteil der angegebenen Verbindung im Sinne eines verlängerten Wirkzeitraums für Dexpanthenol ist.

Besonders bevorzugt sind das Dexpanthenol und das Saccharid über eine glycosidische Bindung chemisch aneinander gebunden. Als glycosidische Bindung bezeichnet man die chemische Bindung zwischen dem anomeren Kohlenstoffatom eines Glycons, also eines Saccharids, und dem Hetero- oder seltener Kohlenstoffatom eines Aglycons, eines "Nichtzuckers". Verbindungen, die eine glycosidische Bindung enthalten, werden allgemein als Glycoside bezeichnet. Die Hydrolyse einer glycosidischen Bindung in einem Glycosid wird insbesondere durch Glucosidasen reversibel katalysiert, wobei das Glycon, vorliegend also das Saccharid, und das Aglycon, hier Dexpanthenol, unter Verbrauch eines Wassermoleküls freigesetzt werden.

Vorzugsweise ist das Saccharid ein Monosaccharid. Monosaccharide sind Einfachzucker mit einem Grundgerüst aus mindestens drei verketteten Kohlenstoffatomen, die eine Carbonylgruppe (-C=O) und mindestens eine Hydroxylgruppe (-OH) aufweisen.

Besonders bevorzugt ist das Monosaccharid ausgewählt aus einer Gruppe, die Pentosen und Hexosen enthält. Hexosen (C₆H₁₂O₆) weisen ein Kohlenstoffgrundgerüst mit sechs Kohlenstoffatomen auf und unterscheiden sich grundsätzlich durch die Art der Carbonylfunktion. Bei einer nicht endständigen Carbonylfunktion (R₁-C(O)-R₂), einer Ketogruppe, spricht man von Ketohexosen. Bei einer endständigen Carbonylfunktion, einer Aldehydgruppe, handelt es sich um Aldohexosen. Pentosen (C₅H₁₀O₅) weisen ein Kohlenstoffgrundgerüst mit fünf Kohlenstoffatomen auf.

Die Hexosen sind vorzugsweise ausgewählt aus einer Gruppe, die α-Glucose, β-Glucose, α-Fructose, β-Fructose, α-Mannose, β-Mannose, α-Galactose und β-Galactose enthält. Je nachdem, welches Saccharid chemisch an das Dexpanthenol gebunden ist, unterscheidet sich auch die Nomenklatur der Verbindung.

Bei einer chemisch an das Dexpanthenol gebundenen α- bzw. β-Glucose handelt es sich bei der Verbindung um α-bzw. β-glycosiliertes Dexpanthenol. Bei einer α-bzw. β-Galactose als chemisch gebundenes Saccharid spricht man von α-bzw. β-galactosyliertem Dexpanthenol. Ist eine α-bzw. β-Fructose als Saccharid an das Dexpanthenol gebunden, handelt es sich um α-bzw. β-fructosyliertes Dexpanthenol.

Vorteilhaft sind die Pentosen ausgewählt aus einer Gruppe, die Arabinose und Xylose enthält.

In einer besonders vorteilhaften Ausgestaltung der Erfindung ist α-Glucose über eine glycosidische Bindung mit Dexpanthenol verbunden. Die Synthese des α-glycosilierten Dexpanthenols erfolgt unter Wasserabspaltung aus α-Glucose und Dexpanthenol Hierbei entsteht α-glycosiliertes Dexpanthenol.

Die Verbindung ist vorzugsweise gekennzeichnet durch die folgende Strukturformel:

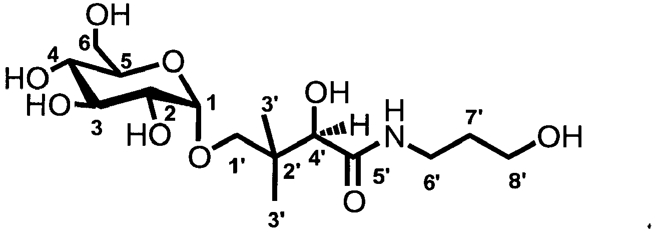

Bei der Synthese des α-glycosilierten Dexpanthenols wird eine glycosidische Bindung zwischen der α-Glucose und dem Dexpanthenol geknüpft. Die Bindung erfolgt besonders vorteilhaft selektiv über die am 1 '-Kohlenstoffatom gebundene Hydroxylgruppe des Dexpanthenols. Das die α-Glucose mit dem Dexpanthenol verbrückende Sauerstoffatom stammt vom Dexpanthenol. Grundsätzlich erfolgt diese Bindungsknüpfung - unabhängig vom eingesetzten Saccharid - vorzugsweise selektiv über die am 1 '-Kohlenstoffatom gebundene Hydroxylgruppe des Dexpanthenols. Für die Selektivität der Bindungsknüpfung ist hierbei ausschließlich das bei der Herstellung des Glykosids eingesetzte Enzym verantwortlich.

Die Verbindung weist eine therapeutische und/oder kosmetische Wirkung auf. Die Verbindung ist insofern bevorzugt zur Verwendung in therapeutischen und/oder kosmetischen Verfahren vorgesehen. Die Verbindung dient hierbei unter verzögerter Freisetzung des Dexpanthenols zur Vorbeugung von Hautschäden und Hautalterung ebenso wie zur Pflege der Haut.

Insbesondere weist die Verbindung eine therapeutische und/oder kosmetische Wirkung als ein Hautpflegeprodukt auf. Bevorzugt ist die Verbindung daher zur Verwendung bei der Hautpflege vorgesehen. Bei protektiver Anwendung über einen längeren Zeitraum wirkt die Verbindung gezielt gegen Hautalterung bzw. beugt dieser vor. Die hydratisierende Wirkung von Dexpanthenol entfaltet sich aufgrund der Wirkzeitverlängerung bzw. der zeitverzögerten Freisetzung über einen längeren Zeitraum. Weiter hat Dexpanthenol bei präventiver Applikation die Fähigkeit, die Haut vor Irritationen zu schützen. Dieser Effekt wird durch eine Verbindung, die Dexpanthenol zeitverzögert freisetzt, verstärkt.

Des Weiteren weist die Verbindung eine vorbeugende Wirkung zur Vermeidung von Hauterkrankungen und/oder zur Behandlung von Hauterkrankungen auf. Vorteilhafterweise ist die Verbindung daher zur Prophylaxe von Hauterkrankungen und/oder zur Behandlung von Hauterkrankungen vorgesehen. Bei konsequenter Anwendung von Dexpanthenol wird durch eine verbesserte Hydratation eine Stabilisierung der Hautbarriere erreicht. Auf diese Weise können durch die Anwendung von Dexpanthenol Hautalterungserscheinungen und Hautveränderungen vorgebeugt werden.

Hierzu zählen beispielsweise ekzematöse Hautveränderungen wie Austrocknungsekzeme und Stauungsekzeme bei Krampfadern, bei Herz- und Niereninsuffizienz, sowie bei Lymphödemen, ebenso wie Kontaktekzeme. Weiterhin kann bakteriellen Infektionen, wie Herpes Zoster und Gürtelrose, Pilzinfektionen wie Candida(Hefepilz)-Mykosen der Haut und der Schleimhäute, sowie Fadenpilzerkrankungen vorgebeugt bzw. diese behandelt werden. Ebenfalls kann Dexpanthenol zur Vorbeugung vor bzw. Behandlung von Hauttumoren, wie beispielsweise menorrhöischen Keratosen ("Alterswarzen"), aktinischen Keratoen (Präkanzerose), Basalzellkarzinomen (Basaliom) und Plattenepithelkarzinomen eingesetzt werden.

Ferner weist die Verbindung eine therapeutische Wirkung zur Beschleunigung der Wundheilung auf. Besonders vorteilhaft ist die Verbindung daher als Medikament zur Beschleunigung der Wundheilung vorgesehen. Im Jahre 1992 veröffentlichten S. Hauptmann, H. Schäfer, A. Fritz und P. Hauptmann in der Zeitschrift "Der Hausarzt: Zeitschrift für Dermatologie, Venerologie und verwandte Gebiete" Untersuchungen hinsichtlich der Wirkung von Dexpanthenol auf die Wundheilung von gingivalen humanen Fibroblasten in einem Konzentrationsbereich von 0,5% bis 10% in vitro. Hierbei wurde festgestellt, dass sich der miotische Index, der Zellteilungsindex, bei allen getesteten Konzentrationen erhöhte.

Die besten Ergebnisse wurden bei der geringsten Konzentration von 0,5% erzielt, die höchste Konzentration von 10 % erwirkte die geringste Wirkung. Im Hinblick auf die Verbindung eines chemisch an ein Saccharid gebundenes Dexpanthenol bedeutet dies, dass nun über einen langen Zeitraum Dexpanthenol bei der topischen Therapie für die Wundheilung und die Zellerneuerung in einer effektiven, also insbesondere einer geringen Konzentration zur Verfügung gestellt wird.

Im Folgenden werden Ausführungsbeispiele der Erfindung anhand von Herstellungsverfahren der Verbindung sowie Zeichnungen näher erläutert. Dabei zeigen:
- Fig. 1: ein exemplarisches ¹H-NMR-Spektrum von α-glycosiliertem Dexpanthenol,
- Fig. 2: ein exemplarisches ¹³C-NMR-Spektrum von α-glycosiliertem Dexpanthenol,
- Fig. 3: ein exemplarisches ¹H-NMR-Spektrum von β-glucosiliertem Dexpanthenol,
- Fig. 4: ein exemplarisches ¹³C-NMR-Spektrum von β-glucosiliertem Dexpanthenol,
- Fig. 5: ein exemplarisches ¹H-NMR-Spektrum von β-galactosilierten Dexpanthenol,
- Fig. 6: ein exemplarisches ¹³C-NMR-Spektrum von β-galactosiliertem Dexpanthenol,
- Fig. 7: ein exemplarisches ¹H-NMR-Spektrum von fructosyliertem Dexpanthenol und
- Fig. 8: ein exemplarisches ¹³C-NMR-Spektrum von fructosyliertem Dexpanthenol.

### (1) Herstellung von α-glycosiliertem Dexpanthenol

Dexpanthenol (0,05 mol/L) und Saccharose (0,4 mol/L) wurden in 0,05 M Phosphatpuffer (0,05 mol/L, pH = 6) gelöst und eine Suspension von Mikroorganismen (*Protaminobacter rubrum* Z 12 (CBS 574.77)) hinzugefügt.

Saccharose besteht aus α-D-Glucose und β-D-Fructose, die über eine α,β-1,2-glycosidische Bindung verknüpft sind. Der Mikroorganismus *Protaminobacter rubrum* Z 12 enthält ein Enzym, eine α-Glucosidase. Es katalysiert die Spaltung der eingesetzten Saccharose in α-D-Glucose und β-D-Fructose. Die α-D-Glucose bindet während der Reaktion chemisch an das Dexpanthenol.

Hierzu wird die Reaktionsmischung bei einer Temperatur von 37 °C im Wasserbad geschüttelt. Nach optimaler Produktbildung wird die Reaktion beendet. Das Produkt α-glycosiliertes Dexpanthenol wurde bei säulenchromatographischen Untersuchungen mit EE-iPrOH-H₂O 6:3:1 als Laufmittel in Form eines weißen Feststoffs erhalten. Die optimale Produktbildung wird durch kontinuierliche Probenentnahme und Dünnschichtchromatographie ermittelt.

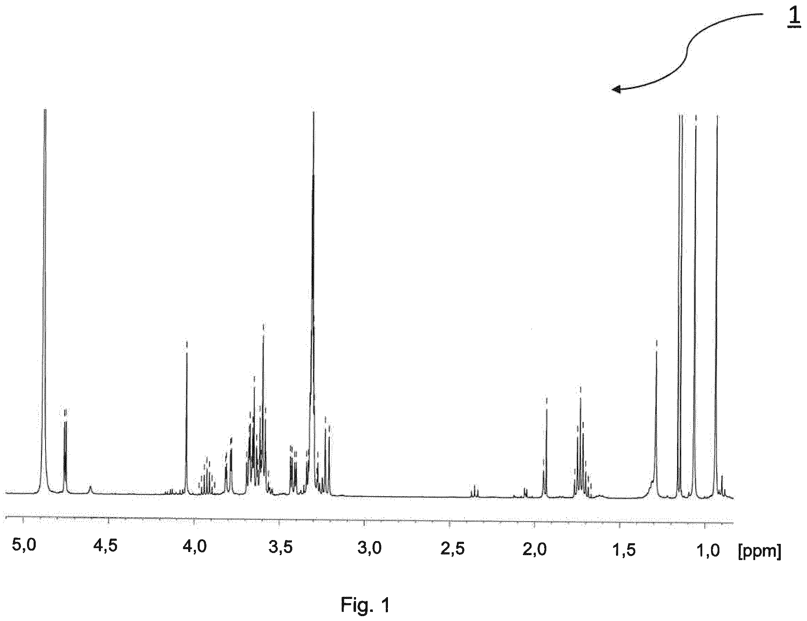

Zum spektroskopischen Nachweis des α-glycosilierten Dexpanthenols wurden ¹H-NMR-Spektren und ¹³C-NMR-Spektren aufgenommen. Fig. 1 zeigt ein exemplarisches ¹H-NMR-Spektrum 1 von α-glycosiliertem Dexpanthenol.

Das ¹H-NMR-Spektrum 1 wurde bei 400 MHz in deuteriertem Methanol aufgenommen, die Zuordnung der Signale ist Tabelle 1 zu entnehmen.

**Tabelle 1: Signalzuordnung ¹H-NMR-Spektrum 1 von α-glycosiliertem Dexpanthenol**

| **δ [ppm]** | **Zuordnung** | **Relatives Integral** | **Multiplizität** | **Kopplungskonstante J [Hz]** |
|---|---|---|---|---|
| 4.76 | H-1 | 1H | Dupplett | 3,7 |
| 4.05 | H-4' | 1H | Singulett | |
| 3.80 | H-6 | 1H | Doppeltes Dupplett | 11,6; 2,2 |
| 3.69-3.58 | H-6b, H-3, H-1'a, H-4, H-8' | 6H | Multiplett | --- |
| 3.42 | H-2 | 1H | Doppeltes Dupplett | 9,6; 3,7 |
| 3.34-3.27 | 3H, H-6', H-5 | 3H | Multiplett | --- |
| 3.22 | H-1'b | 1H | Dupplett | 9.1 |
| 1.77-1.68 | 2H, H-7' | 2H | Multiplett | --- |
| 1.07, 0.94 | H-3'a, H-3'b | 6H | Singulett | --- |

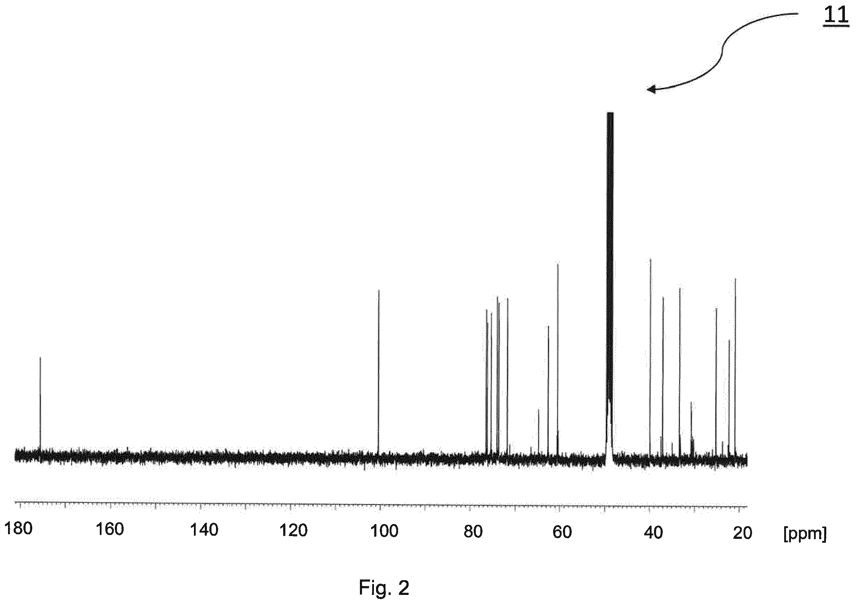

In Fig. 2 ist ein exemplarisches ¹³C-NMR-Spektrum 11 von α-glycosiliertem Dexpanthenol gezeigt. Das ¹³C-NMR-Spektrum 11 wurde bei 100 MHz ebenfalls in deuteriertem Methanol aufgenommen. Die Zuordnung der Signale ist in Tabelle 2 aufgeführt.

**Tabelle 2: Signalzuordnung des ¹³C-NMR-Spektrums 11 von α-glycosiliertem Dexpanthenol**

| **δ [ppm]** | **Zuordnung** |
|---|---|
| 175.65 | C-5' |
| 100.47 | C-1 |
| 76.41 | C-4' |
| 76.14 | C-1' |
| 75.30 | C-3 |
| 73.96 | C-4 |
| 73.59 | C-2 |
| 71.68 | C-5 |
| 62.61 | C-6 |
| 60.54 | C-8' |
| 39.89 | C-2' |
| 37.07 | C-6' |
| 33.33 | C-7' |
| 22.35, 21.00 | C-3'a, C-3'b |

Über die jeweils aus den Tabellen entnehmbaren chemischen Verschiebungen der Signale der H- bzw. C-Atome und der Integralwerte (im Falle der H-Atome) ist eindeutig das α-glucosilierte Dexpanthenol bestimmt.

### (2) Herstellung von β-glucosiliertem Dexpanthenol

Dexpanthenol (0,05 mol/L) und Cellobiose (0,4 mol/L) wurden in Natriumacetatpuffer (0,05 mol/L, pH = 5,2) gelöst und eine β-Glucosidase -Lösung (100 U in Natriumacetatpuffer, β-Glucosidase aus Mandeln, BioChemika 49290, CAS 9001-22-3) hinzugefügt. Die Cellobiose ist ein Disaccharid aus zwei β-1,4-glucosidisch miteinander verknüpften Glucosemolekülen. Das Enzym β-Glucosidase ermöglicht den Abbau der Cellobiose zu Glucose. Die Glucose bindet während der Reaktion chemisch an das Dexpanthenol.

Die Reaktionsmischung wird ebenfalls bei einer Temperatur von 37 °C im Wasserbad geschüttelt. Nach optimaler Produktbildung wird die Reaktion beendet. Das Produkt β-glucosiliertes Dexpanthenol wurde bei säulenchromatographischen Untersuchungen mit EE-iPrOH-H₂O 6:3:1 als Laufmittel in Form eines weißen Feststoffs erhalten.

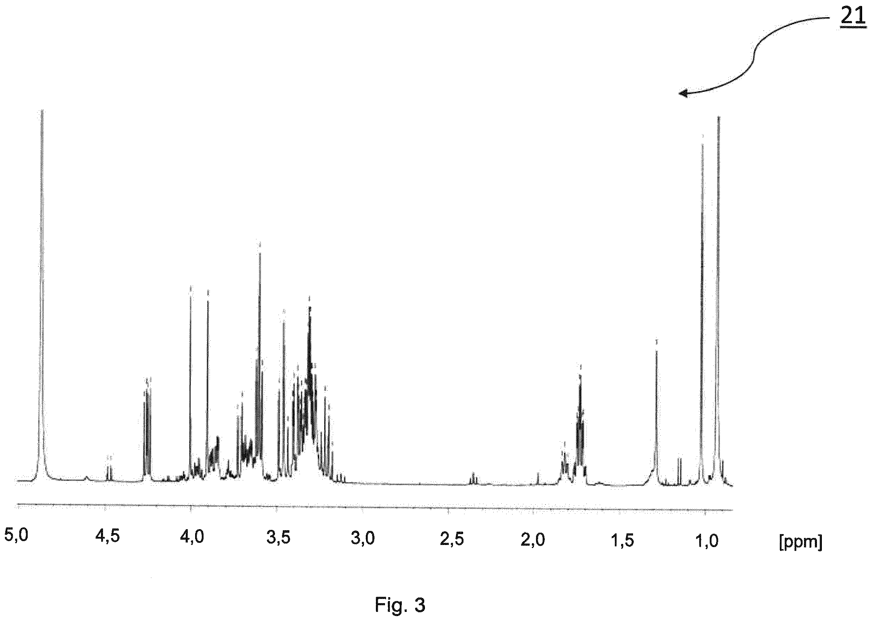

Zum spektroskopischen Nachweis des β-glucosilierten Dexpanthenols wurden ¹H-NMR-Spektren und ¹³C-NMR-Spektren aufgenommen. Fig. 3 zeigt ein exemplarisches ¹H-NMR-Spektrum 21 von β-glucosiliertem Dexpanthenol. Es entstehen bei dieser Reaktion die Glucosylierungsprodukte 2a und 2b.

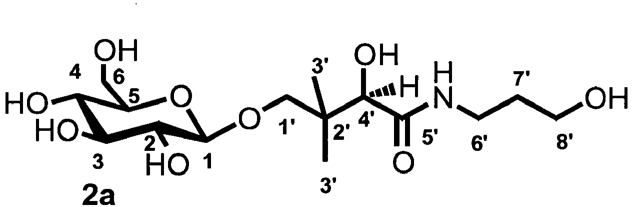

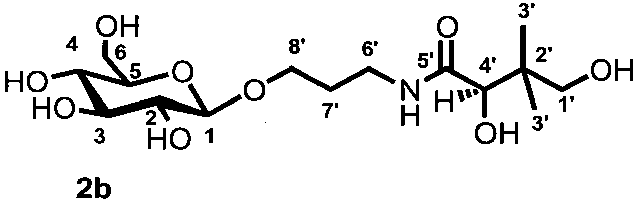

Das ¹H-NMR-Spektrum 21 wurde bei 400 MHz in deuteriertem Methanol aufgenommen, die Zuordnung der Signale ist den folgenden Tabellen 3a und 3b zu entnehmen.

**Tabelle 3a: Signalzuordnung des ¹H-NMR-Spektrums 21 von β-glucosiliertem Dexpanthenol - verknüpft über C-1'O**

| **δ [ppm]** | **Zuordnung** | **Relatives Integral** | **Multiplizität** | **Kopplungskonstante J [Hz]** |
|---|---|---|---|---|
| 4.25 | H-1 | 1H | Dublett | 7,8 |
| 4.01 | H-4' | 1H | Singulett | --- |
| 3.83-3.89, 3.63-3.67 | H-6 | 2H | Multiplett | --- |
| 3.61 | H-8' | 2H | Triplett | 6,2 |
| 3.72,3.44 | H-1' | 2H | zwei Dubletts | 9,1;9,1 |
| 3.20-3.40 | H-3, H-4, H-5 | 3H | Multiplett | --- |
| 3.29-3.36 | H-6' | 2H | Multiplett | --- |
| 3.22 | H-2 | 1H | Multiplett | --- |
| 1.73 | H-7' | 2H | Zwei Quintetts | 6,5;2,1 |
| 1.03, 0.94 | H-3'a, H-3'b | 6H | Singulett | --- |

**Tabelle 3b: Signalzuordnung des ¹H-NMR-Spektrums 21 von β-glucosiliertem Dexpanthenol - verknüpft über C-8'O**

| **δ [ppm]** | **Zuordnung** | **Relatives Integral** | **Multiplizität** | **Kopplungskonstante J [Hz]** |
|---|---|---|---|---|
| 4.26 | H-1 | 1H | Dublett | 7,8 |
| 3.91 | H-4' | 1H | Singulett | --- |
| 3.83-3.89, 3.63-3.67 | H-6 | 2H | Multiplett | --- |
| 3.61-3.65 | H-8' | 2H | Multiplett | --- |
| 3.37-3.50 | H-1' | 2H | Multiplett | --- |
| 3.20-3.40 | H-3, H-4, H-5 | 3H | Multiplett | --- |
| 3.29-3.36 | H-6' | 2H | Multiplett | --- |
| 3.20 | H-2 | 1H | Multiplett | --- |
| 1.73 | H-7' | 2H | Zwei Quintetts | 6,5;2,1 |
| 1.02, 0.94 | H-3'a, H-3'b | 6H | Singulett | --- |

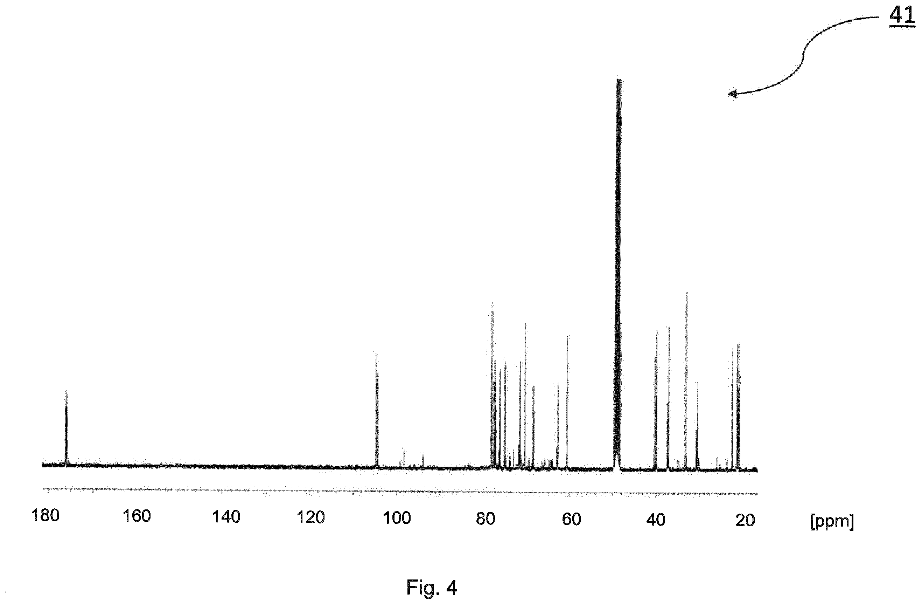

In Fig. 4 ist das ¹³C-NMR-Spektrum 41 von β-glucosiliertem Dexpanthenol gezeigt. Das ¹³C-NMR-Spektrum 41 wurde bei 100 MHz ebenfalls in deuteriertem Methanol aufgenommen. Die Zuordnung der Signale sind in den Tabellen 4a und 4b aufgeführt.

**Tabelle 4a: Signalzuordnung des ¹³C-NMR-Spektrums 41 von β-glucosiliertem Dexpanthenol - verknüpft über C-1'O**

| **δ [ppm]** | **Zuordnung** |
|---|---|
| 176.15 | C-5' |
| 104.72 | C-1 |
| 77.11 | C-1' |
| 76.06 | C-4' |
| 77.95 | C-3 |
| 77.93 | C-4 |
| 75.01 | C-2 |
| 71.57 | C-5 |
| 60.56 | C-8' |
| 60.52 | C-6 |
| 39.91 | C-2' |
| 37.03 | C-6' |
| 33.19 | C-7' |
| 22.34, 21.31 | C-3'a, C-3'b |

**Tabelle 4b: Signalzuordnung des ¹³C-NMR-Spektrums 41 von β-glucosiliertem Dexpanthenol - verknüpft über C-8'O**

| **δ [ppm]** | **Zuordnung** |
|---|---|
| 175.94 | C-5' |
| 104.32 | C-1 |
| 78.02 | C-3 |
| 77.39 | C-4' |
| 77.31 | C-4 |
| 75.05 | C-2 |
| 71.52 | C-5 |
| 70.34 | C-1' |
| 68.43 | C-8' |
| 62.62 | C-6 |
| 40.34 | C-2' |
| 37.27 | C-6' |
| 30.39 | C-7' |
| 21.36, 20.83 | C-3'a, C-3'b |

Über die jeweils aus den Tabellen entnehmbaren chemischen Verschiebungen der Signale der H- bzw. C-Atome und der Integralwerte (im Falle der H-Atome) ist eindeutig das β-glucosilierte Dexpanthenol bestimmt.

### (3) Herstellung von β-galactosyliertem Dexpanthenol

Dexpanthenol (0,05 mol/L) und Lactose (0,4 mol/L) wurden in Natriumacetatpuffer(0,05 mol/L, pH = 5,2) gelöst und eine β-Galactosidase-Lösung (100 U in Natriumacetatpuffer, β-Galactosidase aus *Aspergillus oryzae,* Sigma G5160, CAS 9031-11-2) hinzugefügt. Lactose besteht aus den D-Galactose und D-Glucose, die über eine β-1,4-glycosidische Bindung verbunden sind. β-Galactosidasen katalysiert die Hydrolyse dieser Bindung enzymatisch, wobei Galactose entsteht, die während der Reaktion chemisch an das Dexpanthenol bindet.

Die Reaktionsmischung wird hierzu bei einer Temperatur von 37 °C im Wasserbad geschüttelt. Nach optimaler Produktbildung wird die Reaktion beendet. Das Produkt β-galactosyliertes Dexpanthenol wurde bei säulenchromatographischen Untersuchungen mit EE-iPrOH-H₂O 6:3:1 als Laufmittel in Form eines weißen Feststoffs erhalten.

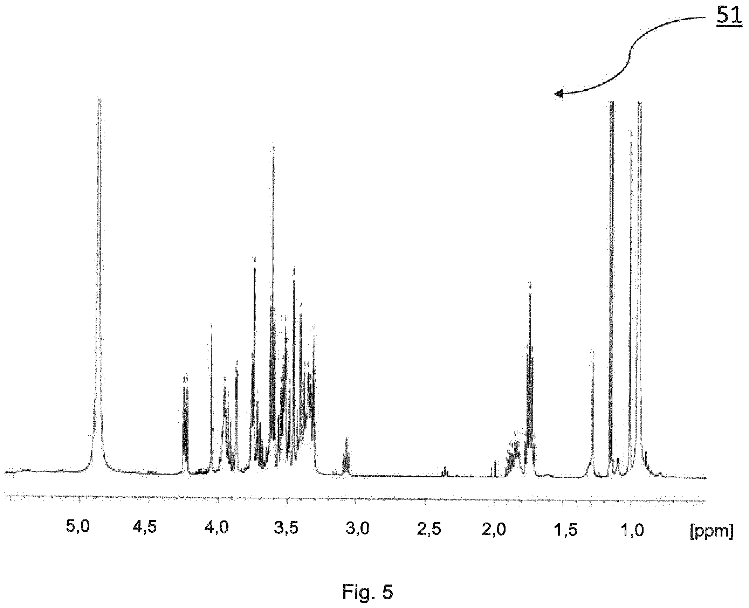

Zum spektroskopischen Nachweis des β-galactosilierten Dexpanthenols wurden ¹H-NMR-Spektren und ¹³C-NMR-Spektren aufgenommen. Fig. 5 zeigt ein exemplarisches ¹H-NMR-Spektrum von β-galactosilierten Dexpanthenol. Es entstehen bei dieser Reaktion die nachfolgend gezeigten Galactosylierungsprodukte 3a und 3b.

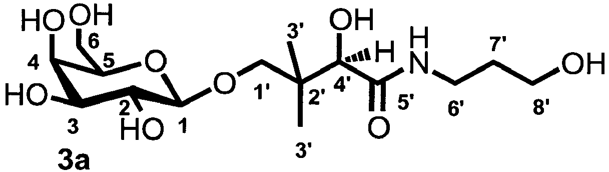

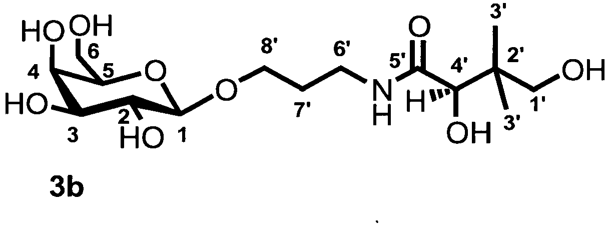

Das ¹H-NMR-Spektrum 51 wurde bei 400 MHz in deuteriertem Methanol aufgenommen, die Zuordnung der Signale ist den Tabellen 5a und 5b zu entnehmen.

**Tabelle 5a: Signalzuordnung des ¹H-NMR-Spektrums 51 von β-galactosiliertem Dexpanthenol - verknüpft über C-1'O**

| **δ [ppm]** | **Zuordnung** | **Relatives Integral** | **Multiplizität** | **Kopplungskonstante J [Hz]** |
|---|---|---|---|---|
| 4.24 | H-1 | 1H | Dublett | 7,8 |
| 4.05 | H-4' | 1H | Singulett | --- |
| 3.92-3.97 | H-5 | 1H | Multiplett | --- |
| 3.86-3.88 | H-3 | 1H | Multiplett | --- |
| 3.74-3.76 | H-6 | 2H | Multiplett | --- |
| 3.74-3.76, 3.51-3.56 | H-1' | 2H | Multiplett | --- |
| 3.61 | H-8' | 2H | Triplett | 6,2 |
| 3.51 | H-2 | 1H | Multiplett | --- |
| 3.48-3.51 | H-4 | 1H | Multiplett | --- |
| 3.31-3.37 | H-6' | 2H | Multiplett | --- |
| 1.74 | H-7' | 2H | Quintett | 6,4 |
| 1.01, 0.95 | H-3'a, H-3'b | 6H | Singulett | --- |

**Tabelle 5b: Signalzuordnung des ¹H-NMR-Spektrums 51 von β-galactosiliertem Dexpanthenol - verknüpft über C-8'O**

| **δ [ppm]** | **Zuordnung** | **Relatives Integral** | **Multiplizität** | **Kopplungskonstante J [Hz]** |
|---|---|---|---|---|
| 4.25 | H-1 | 1H | Dublett | 7,8 |
| 3.95 | H-4' | 1H | Singulett | --- |
| 3.92-3.97 | H-5 | 1H | Multiplett | --- |
| 3.86-3.88 | H-3 | 1H | Multiplett | |
| 3.74-3.76 | H-6 | 2H | Multiplett | |
| 3.63, 3.96 | H-8' | 2H | Multiplett | --- |
| 3.51-3.56 | H-2 | 1H | Multiplett | --- |
| 3.48-3.52 | H-4 | 1H | Multiplett | |
| 3.39, 3.47 | H-1' | 2H | 2 Dubletts | 11,1; 11,4 |
| 3.35 | H-6' | 2H | Multiplett | |
| 1.83 | H-7' | 2H | Multiplett | |
| 1.01, 0.95 | H-3'a, H-3'b | 6H | Singulett | --- |

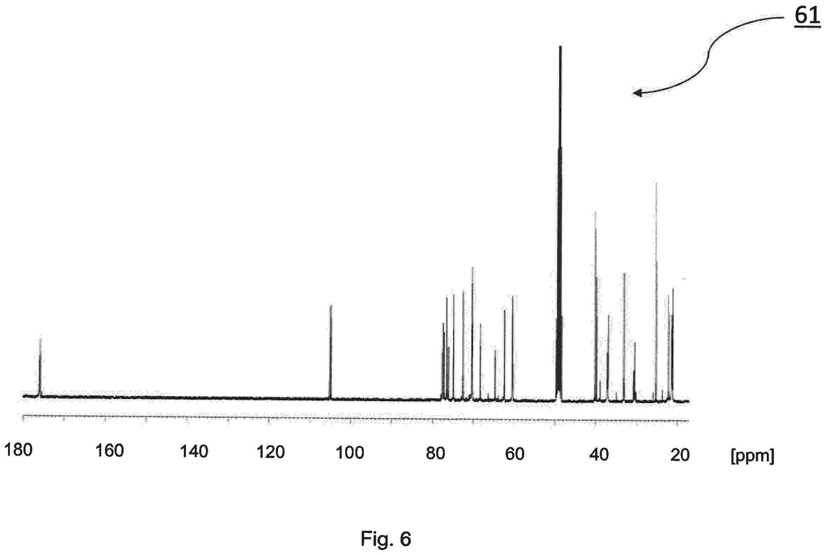

In Fig. 6 ist das ¹³C-NMR-Spektrum 61 von β-galactosiliertem Dexpanthenol gezeigt. Das ¹³C-NMR-Spektrum 61 wurde bei 100 MHz ebenfalls in deuteriertem Methanol aufgenommen. Die Zuordnung der Signale ist in den Tabellen 6a und 6b aufgeführt.

**Tabelle 6a: Signalzuordnung des ¹³C-NMR-Spektrums 61 von β-galactosiliertem Dexpanthenol - verknüpft über C-1'O**

| **δ [ppm]** | **Zuordnung** |
|---|---|
| 176.15 | C-5' |
| 105.20 | C-1 |
| 77.44 | C-5 |
| 76.97 | C-1' |
| 76.52 | C-2 |
| 76.44 | C-5 |
| 76.11 | C-4' |
| 70.30 | C-3 |
| 62.40 | C-6 |
| 60.37 | C-8' |
| 39.84 | C-2' |
| 37.01 | C-6' |
| 33.08 | C-7' |
| 22.11, 21.15 | C-3'a, C-3'b |

**Tabelle 6b: Signalzuordnung des ¹³C-NMR-Spektrums 61 von β-galactosiliertem Dexpanthenol - verknüpft über C-8'O**

| **δ [ppm]** | **Zuordnung** |
|---|---|
| 175.92 | C-5' |
| 104.82 | C-1 |
| 77.51 | C-4' |
| 77.44 | C-5 |
| 76.46 | C-4 |
| 72.46 | C-2 |
| 70.35 | C-3 |
| 70.21 | C-1' |
| 68.30 | C-8' |
| 62.39 | C-6 |
| 40.27 | C-2' |
| 37.03 | C-6' |
| 30.45 | C-7' |
| 21.31, 21.40 | C-3'a, C-3'b |

Über die jeweils aus den Tabellen entnehmbaren chemischen Verschiebungen der Signale der H- bzw. C-Atome und der Integralwerte (im Falle der H-Atome) ist eindeutig das β-galactosilierte Dexpanthenol bestimmt.

### (4) Herstellung von fructosyliertem Dexpanthenol

Dexpanthenol (0,05 mol/L) und Saccharose (0,4 mol/L) wurden in Natriumacetatpuffer (0,05 mol/L, pH = 5,2) gelöst und eine Fructosidase-Lösung (100 U in Natriumacetatpuffer, Boehringer Mannheim GmbH, β-Fructosidase aus Hefe Bestellnummer 104914) hinzugefügt. Die Fructosidase-Lösung katalysiert die Spaltung der Saccharose in α-D-Glucose und β-D-Fructose enzymatisch. Die β-D-Fructose bindet während der Reaktion chemisch an das Dexpanthenol.

Die Reaktionsmischung wird hierzu ebenfalls bei einer Temperatur von 37 °C im Wasserbad geschüttelt. Nach optimaler Produktbildung wird die Reaktion beendet. Das Produkt fructosyliertes Dexpanthenol wurde bei säulenchromatographischen Untersuchungen mit EE-iPrOH-H₂O 6:3:1 als Laufmittel in Form eines weißen Feststoffs erhalten. Das Produkt fructosyliertes Dexpanthenol hat die Strukturformel

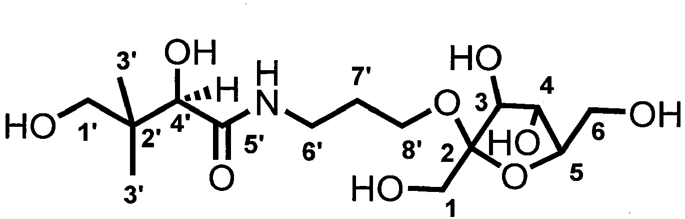

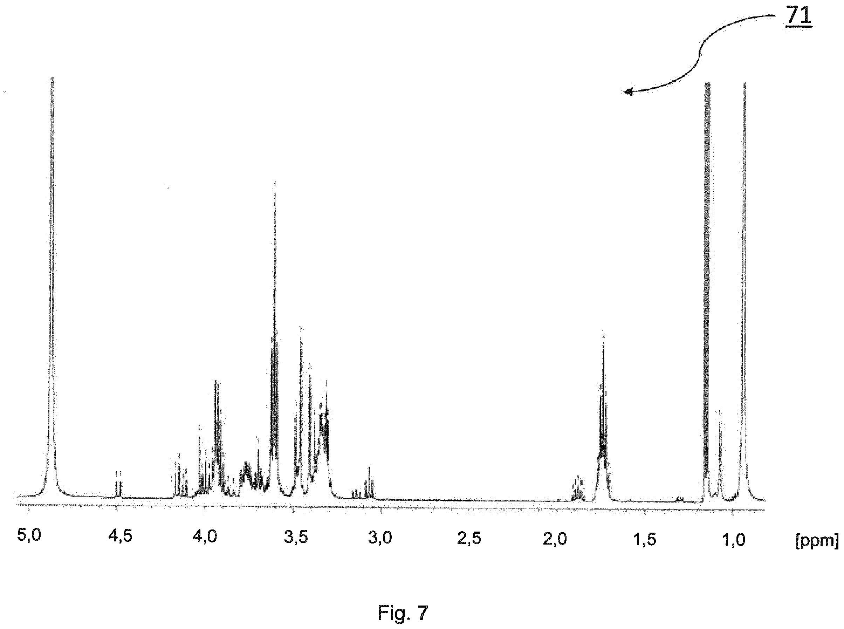

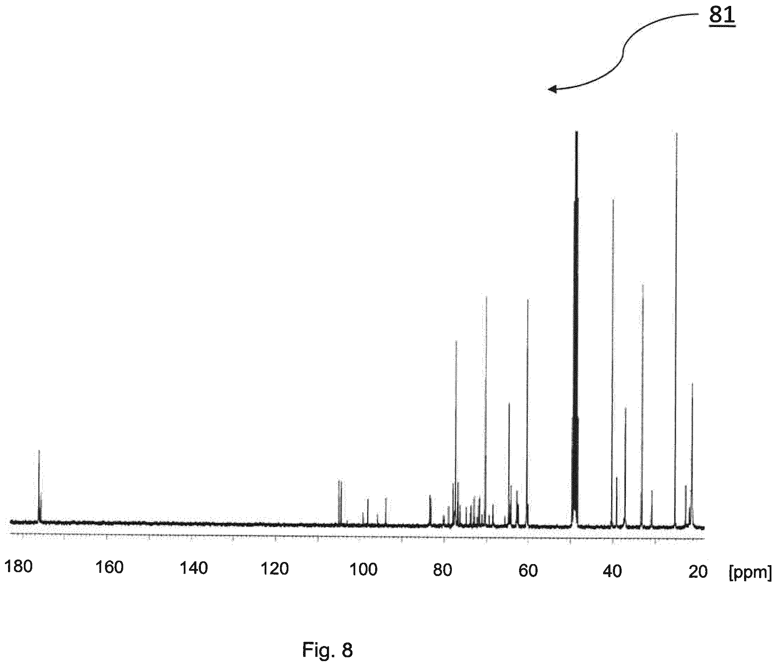

Zum spektroskopischen Nachweis des fructosylierten Dexpanthenols wurden ebenfalls ¹H-NMR-Spektren und ¹³C-NMR-Spektren aufgenommen. Fig. 7 zeigt ein exemplarisches ¹H-NMR-Spektrum 71 von fructosyliertem Dexpanthenol. In Fig. 8 ist ein exemplarisches ¹³C-NMR-Spektrum 81 von fructosyliertem Dexpanthenol gezeigt. Anhand der Signale aus den Spektren 71, 81 ist das konjugierte Produkt aus Fruktose und Dexpanthenol zu entnehmen. Die Zuordnung der Signale ist den Tabellen 7 bzw. 8 entnehmbar.

**Tabelle 7**

| **δ [ppm]** | **Zuordnung** | **Relatives Integral** | **Multiplizität** | **Kopplungskonstante J [Hz]** |
|---|---|---|---|---|
| 4.16 | H-3 | 1H | Dublett | 8.9 |
| 4.03 | H-4' | 1H | Singulett | |
| 4.00 | H-4 | 1H | Triplett | 8.9 |
| 3.76 | H-5 | 1H | Multiplett | |
| 3.61 | H-1, H-6, H-8' | 6H | Multiplett | |
| 3.47 | H-1' | 2H | Multiplett | |
| 3.34 | H-6' | 2H | Multiplett | |
| 1.73 | H-7' | 2H | Multiplett | |
| 1.07 | H-3' | 6H | Singulett | |

**Tabelle 8**

| **δ [ppm]** | **Zuordnung** |
|---|---|
| 175.65 | C-5' |
| 104.43 | C-2 |
| 83.52 | C-5 |
| 80.07 | C-3 |
| 77.44 | C-4' |
| 76.67 | C-4 |
| 77.44 | C-4' |
| 68.40 | C-1' |
| 64.17 | C-6 |
| 63.80 | C-1 |
| 60.59 | C-8' |
| 39.13 | C-2' |
| 37.03 | C-6' |
| 33.14 | C-7' |
| 20.74 | 2 x C-3' |

Über die jeweils aus den Tabellen 7 und 8 entnehmbaren chemischen Verschiebungen der Signale der H- bzw. C-Atome und der Integralwerte (im Falle der H-Atome) ist eindeutig das fructosylierte Dexpanthenol bestimmt.

### Bezugszeichenliste

- 1: ¹H-NMR-Spektrum von α-glucosiliertem Dexpanthenol
- 11: ¹³C-NMR-Spektrum von α-glucosilierten Dexpanthenol
- 21: ¹H-NMR-Spektrum von β-glucosilierten Dexpanthenol
- 41: ¹³C-NMR-Spektrum von β-glucosilierten Dexpanthenol
- 51: ¹H-NMR-Spektrum von β-galactosilierten Dexpanthenol
- 61: ¹³C-NMR-Spektrum von β-galactosilierten Dexpanthenol
- 71: ¹H-NMR-Spektrum von β-fructosilierten Dexpanthenol
- 81: ¹³C-NMR-Spektrum von β-fructosilierten Dexpanthenol

## Patentansprüche

1. Verbindung, **gekennzeichnet durch** ein chemisch an ein Saccharid gebundenes Dexpanthenol.

2. Verbindung nach Anspruch 1, wobei das Dexpanthenol und das Saccharid über eine glycosidische Bindung chemisch aneinander gebunden sind.

3. Verbindung nach Anspruch 1 oder 2, wobei das Saccharid ein Monosaccharid ist.

4. Verbindung nach Anspruch 3, wobei das Monosaccharid ausgewählt ist aus einer Gruppe, die Hexosen und Pentosen enthält.

5. Verbindung nach Anspruch 4, wobei die Hexosen ausgewählt sind aus einer Gruppe, die α-Glucose, β-Glucose, α-Fructose, β-Fructose, α-Galactose und β-Galactose, α-Mannose und β-Mannose enthält.

6. Verbindung nach Anspruch 4 oder 5, wobei die Pentosen ausgewählt sind aus einer Gruppe, die Arabinose und Xylose enthält.

7. Verbindung nach Anspruch 5 oder 6, wobei α-Glucose über eine glycosidische Bindung mit Dexpanthenol verbunden ist.

8. Verbindung nach einem der vorhergehenden Ansprüche, **gekennzeichnet durch** folgende Strukturformeln:

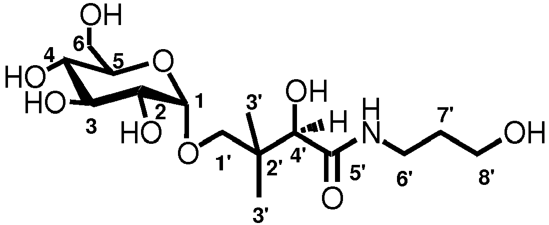

9. Verbindung nach einem der Ansprüche 1 bis 8 zur Verwendung in therapeutischen und/oder kosmetischen Verfahren.

10. Verbindung nach einem der Ansprüche 1 bis 9 zur Verwendung bei der Hautpflege.

11. Verbindung nach einem der Ansprüche 1 bis 9 zur Verwendung bei der Prophylaxe von Hauterkrankungen und/oder bei der Behandlung von Hauterkrankungen.

12. Verbindung nach einem der Ansprüche 1 bis 9 zur Verwendung als Medikament zur Beschleunigung der Wundheilung.

## Claims

1. Compound, **characterized by** a dexpanthenol chemically bonded to a saccharide.

2. Compound according to Claim 1, wherein the dexpanthenol and the saccharide are chemically bonded to one another via a glycosidic bond.

3. Compound according to Claim 1 or 2, wherein the saccharide is a monosaccharide.

4. Compound of Claim 3, wherein the monosaccharide is selected from a group containing hexoses and pentoses.

5. Compound according to Claim 4, wherein the hexoses are selected from a group comprising α-glucose, β-glucose, α-fructose, β-fructose, α-galactose and β-galactose, α-mannose and β-mannose.

6. Compound according to Claim 4 or 5, wherein the pentoses are selected from a group containing arabinose and xylose.

7. Compound according to Claim 5 or 6, wherein α-glucose is bonded to dexpanthenol via a glycosidic bond.

8. Compound according to one of the aforementioned Claims, **characterized by** the following structural formulas:

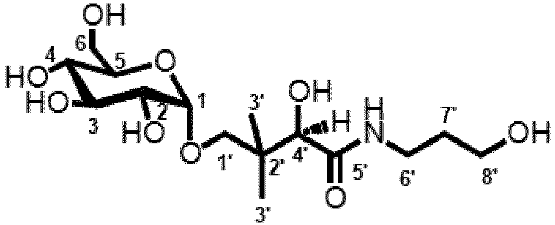

9. Compound according to any of Claims 1 to 8 for use in therapeutic and / or cosmetic methods.

10. Compound according to any of Claims 1 to 9 for use in skin care.

11. Compound according to any of Claims 1 to 9 for use in the prophylaxis of skin diseases and / or in the treatment of skin diseases.

12. Compound according to any of Claims 1 to 9 for use as medication to promote wound healing.

## Revendications

1. Composé, **caractérisé par** un dexpanthénol lié chimiquement à un saccharide.

2. Composé selon la revendication 1, dans lequel le dexpanthénol et le saccharide sont liés chimiquement l'un à l'autre via une liaison glycosidique.

3. Composé selon la revendication 1 ou 2, dans lequel le saccharide est un monosaccharide.

4. Composé selon la revendication 3, dans lequel le monosaccharide est sélectionné parmi un groupe contenant des hexoses et des pentoses.

5. Composé selon la revendication 4, dans lequel les hexoses sont sélectionnés parmi un groupe contenant de la α-glucose, β-glucose, α-fructose, β-fructose, α-galactose et β-galactose, α-mannose et β-mannose.

6. Composé selon la revendication 4 ou 5, dans lequel les pentoses sont sélectionnés parmi un groupe contenant de l'arabinose et du xylose.

7. Composé selon la revendication 5 ou 6, dans lequel l'α-glucose est lié au dexpanthénol via une liaison glycosidique.

8. Composé selon l'une des revendications précédentes, **caractérisée par** les formules structurelles suivantes:

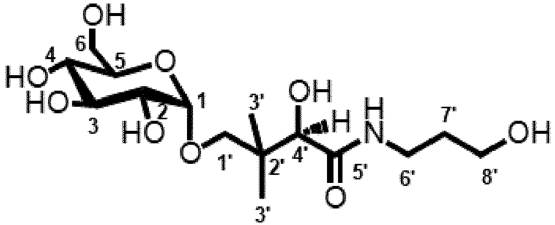

9. Composé selon l'une des revendications 1 à 8, destiné à l'utilisation dans des méthodes thérapeutiques et / ou cosmétiques.

10. Composé selon l'une des revendications 1 à 9 destiné à l'utilisation dans les soins de la peau.

11. Composé selon l'une des revendications 1 à 9 destiné à l'utilisation dans la prophylaxie des maladies de la peau et / ou dans le traitement des maladies de la peau.

12. Composé selon l'une des revendications 1 à 9 destiné à l'utilisation comme médicament pour favoriser la cicatrisation des plaies.
